# EUROPÄISCHE PATENTSCHRIFT

(11) **EP 4 470 509 B1**
(45) Veröffentlichungstag und Bekanntmachung des Hinweises auf die Patenterteilung: **28.01.2026**
(21) Anmeldenummer: 24170683.7
(22) Anmeldetag: 17.04.2024
(51) Int. Cl.: A61F 2/36, A61F 2/38

(54) **PROTHESE, INSBESONDERE KNIEPROTHESE ODER HÜFTSCHAFTPROTHESE**
PROSTHESIS, IN PARTICULAR KNEE PROSTHESIS OR HIP STEM PROSTHESIS
PROTHÈSE, EN PARTICULIER PROTHÈSE DE GENOU OU PROTHÈSE DE TIGE FÉMORALE

(30) Priorität: 02.06.2023 DE 102023114598
(43) Veröffentlichungstag der Anmeldung: 04.12.2024
(73) Patentinhaber: Aesculap AG, 78532 Tuttlingen (DE)
(72) Erfinder: Kempf, Harry, 72510 Stetten am kalten Markt (DE)
(74) Vertreter: DREISS Patentanwälte PartG mbB

(56) Entgegenhaltungen:
- US-A- 4 846 839
- US-A1- 2006 167 555
- US-A1- 2008 027 559
- US-A1- 2014 277 536

## Beschreibung

Die Erfindung betrifft eine Prothese, insbesondere Knieprothese oder Hüftschaftprothese, mit einem Schaft zur Anordnung und Befestigung in einem Röhrenknochen, wobei der Schaft sich längs einer Schaftachse zwischen einem proximalen Ende und einem in den Röhrenknochen einführbaren distalen Ende erstreckt, wobei der Schaft von dem proximalen Ende ausgehend entlang einer ersten Teillänge einen ersten, insbesondere massiven, Querschnitt aufweist und wobei der Schaft entlang einer zweiten Teillänge, welche das distale Ende des Schafts einschließt, einen von dem ersten Querschnitt abweichenden zweiten Querschnitt aufweist, wobei der Schaft entlang der zweiten Teillänge sich parallel zu der Schaftachse erstreckende Schaftabschnitte aufweist, welche durch einen schlitzförmigen Zwischenraum voneinander getrennt sind, wobei die Schaftabschnitte ausgehend von einem unverformten Ruhezustand durch Biegebelastung derart verformbar sind, dass sich der schlitzförmige Zwischenraum verkleinert.

Prothesen der vorstehenden Art sind beispielsweise aus der EP 0 966 928 A2 bekannt. Zur Befestigung des Schafts in einem Röhrenknochen wird das distale Ende des Schafts in einen Knochenmarkraum des Röhrenknochens eingeführt. Dabei hat der schlitzförmige Zwischenraum den Zweck, dass sich die Schaftabschnitte im Zuge ihrer Einführung in den Knochenmarkraum elastisch und nach radial innen verformen können, wobei sich der schlitzförmige Zwischenraum verkleinert. Die Schaftabschnitte liegen nach vollständiger Einführung in den Knochenmarkraum unter Vorspannung an einer Begrenzung des Knochenmarkraums an.

Es hat sich herausgestellt, dass infolge dieser Vorspannung zumindest bei einigen Patienten auch längere Zeit nach Einsatz der Prothese ein sogenannter Schaftschmerz auftreten kann. Es besteht daher Bedarf, Schaftabschnitte bereitzustellen, welche mit niedrigen Kräften verformbar sind, um auf diese Weise die elastischen Rückstellkräfte zu reduzieren. Gleichzeitig besteht aber das Problem, dass eine zu labile Ausbildung der Schaftabschnitte mit einer unerwünschten plastischen Verformung oder sogar einem Versagen der Schaftabschnitte einhergehen könnte.

Hiervon ausgehend liegt der vorliegenden Erfindung die Aufgabe zugrunde, eine Prothese bereitzustellen, welche möglichst niedrige Rückstellkräfte entwickelt, aber gleichzeitig eine genügend hohe Stabilität aufweist.

Diese Aufgabe wird bei einer Prothese der eingangs genannten Art erfindungsgemäß dadurch gelöst, dass ein erster Schaftabschnitt einen ersten Vorsprung aufweist, der auf einer einem zweiten Schaftabschnitt zugewandten Seite des ersten Schaftabschnitt vorgesehen ist, eine erste Verengung des Zwischenraums bildet und entlang der Schaftachse gesehen zu der ersten Teillänge des Schafts beabstandet angeordnet ist, wobei der erste Vorsprung eine erste Begrenzungsfläche zur Anlage an dem zweiten Schaftabschnitt aufweist, wobei die erste Begrenzungsfläche in dem unverformten Ruhezustand zu dem zweiten Schaftabschnitt beabstandet ist.

Bei der erfindungsgemäßen Prothese ist mindestens ein erster Schaftabschnitt vorgesehen, dessen maximale Biegeverformung durch einen Vorsprung begrenzt ist, der eine erste Begrenzungsfläche aufweist, die in einem maximal verformten Zustand des ersten Schaftabschnitts an dem zweiten Schaftabschnitt anliegt. In einem unverformten Ruhezustand ist der Querschnitt des schlitzförmigen Zwischenraums im Bereich des ersten Vorsprungs verengt. In diesem unverformten Ruhezustand des ersten Schaftabschnitts ist die erste Begrenzungsfläche des Vorsprungs zu dem zweiten Schaftabschnitt beabstandet. Dies ermöglicht es, dass sich der erste Schaftabschnitt bei einer Biegebelastung auch längs der Schaftachse gesehen auf Höhe des ersten Vorsprungs verformen kann. Der Verformungsweg des ersten Schaftabschnitts auf Höhe des ersten Vorsprungs ist allerdings auf einen Zustand begrenzt, in welchem die erste Begrenzungsfläche des ersten Vorsprungs des ersten Schaftabschnitts in Anlage mit dem zweiten Schaftabschnitt gelangt.

Es ist insbesondere möglich, dass der erste Schaftabschnitt ein vergleichsweise kleines Flächenträgheitsmoment aufweist, also bei dem operativen Einsetzen der Prothese in den Röhrenknochen mit nur niedrigen Verformungskräften verformbar ist und nach dem Einsetzen in den Knochenmarkraum vergleichsweise niedrige Rückstellkräfte entwickelt. Gleichwohl kann eine unerwünschte plastische Verformung oder sogar ein Versagen mit Bruch des ersten Schaftabschnitts verhindert werden, da der erste Schaftabschnitt sich in Folge der Anlage der ersten Begrenzungsfläche an den zweiten Schaftabschnitt nicht weiter verformen kann. Der maximale Verformungsweg des ersten Schaftabschnitts ist durch das Maß begrenzt, mit welchem der Vorsprung den schlitzförmigen Zwischenraum verengt. Dieses Maß ist durch die Höhe des ersten Vorsprungs definiert, der sich über eine Grundebene des ersten Schaftabschnitts hinausgehend in Richtung auf den zweiten Schaftabschnitt erstreckt.

Die erfindungsgemäße Prothese ermöglicht die Verwendung von Materialien, die sich in der Praxis bewährt haben, also beispielsweise von Titan- oder Stahllegierungen.

Besondere Vorteile ergeben sich, wenn der zweite Schaftabschnitt einen zweiten Vorsprung aufweist, der auf einer dem ersten Schaftabschnitt zugewandten Seite des zweiten Schaftabschnitts vorgesehen ist, eine zweite Verengung des Zwischenraums bildet und entlang der Schaftachse gesehen zu der ersten Teillänge des Schafts beabstandet angeordnet ist, wobei der zweite Vorsprung eine zweite Begrenzungsfläche zur Anlage an dem ersten Schaftabschnitt aufweist, wobei die zweite Begrenzungsfläche in dem unverformten Ruhezustand zu dem ersten Schaftabschnitt beabstandet ist.

Der erste Schaftabschnitt und der zweite Schaftabschnitt sind vorzugsweise auf einander gegenüberliegenden Seiten einer zentralen Schaftachse angeordnet. Wenn auch der zweite Schaftabschnitt mit einem zweiten Vorsprung versehen ist, kann der Verformungsweg auch des zweiten Schaftabschnitts dadurch begrenzt werden, dass die zweite Begrenzungsfläche des zweiten Vorsprungs an dem ersten Schaftabschnitt zur Anlage kommt.

Es ist möglich, dass der erste Vorsprung und der zweite Vorsprung längs der Schaftachse gesehen zueinander versetzt angeordnet sind, sodass bei einer Biegebelastung zunächst eine der beiden Begrenzungsflächen mit einem der beiden Schaftabschnitte in Anlage gelangt und sodass erst nachfolgend die andere Begrenzungsfläche mit dem anderen Schaftabschnitt in Anlage gelangt.

Es ist aber auch möglich, dass der erste Vorsprung und der zweite Vorsprung längs der Schaftachse gesehen auf derselben Höhe angeordnet sind und dass eine Biegeverformung der Schaftabschnitte im Bereich des ersten Vorsprungs und des zweiten Vorsprungs durch eine Anlage der ersten Begrenzungsfläche und der zweiten Begrenzungsfläche begrenzt ist. Dies ist insbesondere vorteilhaft, wenn die Schaftabschnitte bezogen auf die Schaftachse zueinander spiegelsymmetrische Querschnitte aufweisen, was zur Folge hat, dass sich die beiden Schaftabschnitte bei Einführung des distalen Endes in einen Röhrenknochen bezogen auf die Schaftachse symmetrisch verformen und im eingesetzten Zustand der Prothese einander entgegengesetzte und betragsgleiche Rückstellkräfte auf einander zugewandte Abschnitte einer Begrenzung des Knochenmarkraums ausüben.

Es ist ferner bevorzugt, wenn in dem unverformten Ruhezustand der Schaftabschnitte oder in einem verformten Zustand der Schaftabschnitte die erste Begrenzungsfläche und die zweite Begrenzungsfläche zueinander parallel orientiert sind. Bei einer parallelen Orientierung der Begrenzungsflächen in dem unverformten Ruhezustand der Schaftabschnitte und einer Verformung der Schaftabschnitte gelangen die beiden Begrenzungsflächen initial zunächst nur entlang einer Berührlinie miteinander in Kontakt. Bei einer weiteren Verformung erweitert sich diese Berührlinie bis hin zu einem Zustand, in welchem die Begrenzungsflächen über ihre Erstreckung hinweg (also flächig) aneinander anliegen. In diesem Zustand können die beiden Schaftabschnitte stabil gegeneinander abgestützt werden und zwar unter Vermeidung lokal erhöhter Flächenpressungen. Dies gilt insbesondere, wenn die Begrenzungsflächen in einem unverformten Ruhezustand zueinander leicht winklig angeordnet sind, wobei der Winkel derart gewählt ist, dass die beiden Begrenzungsflächen in dem maximal verformten Zustand der Schaftabschnitte gleichzeitig und über ihre gesamte Erstreckung hinweg miteinander in Anlage gelangen.

Besonders bevorzugt ist es, dass sich die erste Begrenzungsfläche und/oder die zweite Begrenzungsfläche längs der Schaftachse gesehen bis zu dem distalen Ende des Schafts erstreckt oder erstrecken. Auf diese Weise kann ein Verformungsweg eines Schaftabschnitts oder der Schaftabschnitte in einem Bereich der Schaftabschnitte begrenzt werden, der bei einer Biegebelastung einen maximalen Verformungsweg aufweist. Außerdem ist es vorteilhaft, dass der schlitzförmige Zwischenraum an dem distalen Ende des Schafts einen in Folge der Vorsprünge verengten Querschnitt aufweist, wodurch das distale Ende besonders einfach in einen Knochenmarkraum einführbar ist.

Es wird ferner vorgeschlagen, dass die erste Begrenzungsfläche und/oder die zweite Begrenzungsfläche längs der Schaftachse gesehen eine Begrenzungsflächenlänge aufweist oder aufweisen, welche zwischen 5% und 45%, insbesondere zwischen 15% und 35%, der zweiten Teillänge beträgt. Mit anderen Worten: 95% bis 55%, insbesondere 85% bis 65%, der zweiten Teillänge der Schaftabschnitte weist vorzugsweise keine Vorsprünge auf, also einen schlitzförmigen Zwischenraum, der nicht mittels mindestens eines Vorsprungs verengt ist. Diese "vorsprungfreie" zweite Teillänge der Schaftabschnitte ermöglicht die Bereitstellung eines großvolumigen Zwischenraums, der mit einer entsprechenden Verkleinerung des Querschnitts der Schaftabschnitte einhergeht, welche dadurch niedrige Verformungswiderstände aufweisen.

Die Schaftabschnitte weisen in einem sich an die erste Teillänge des Schafts anschließenden Bereich der zweiten Teillänge des Schafts und in dem unverformten Ruhezustand der Schaftabschnitte einen der Breite des schlitzförmigen Zwischenraums entsprechenden Schaftabschnittsabstand auf. Dieser ("vorsprungfreie") Schaftabschnittsabstand beträgt vorzugsweise mindestens 20%, insbesondere mindestens 25%, eines maximalen Durchmessers einer Umfangsfläche des Schafts. Dieser maximale Durchmesser beträgt beispielsweise 10 mm, 12 mm, 14 mm, 16 mm, 18 mm, 20 mm, 22 mm oder 24 mm. Für das Beispiel eines Schafts mit einem Durchmesser von 10 mm ist es also bevorzugt, dass der genannte Schaftabschnittsabstand mindestens 2 mm beträgt, sodass die radiale Erstreckung der Schaftabschnitte bei symmetrischer Ausbildung relativ zu der Schaftachse maximal 4 mm beträgt.

Der erste Vorsprung weist eine senkrecht zu der ersten Begrenzungsfläche gemessene erste Höhe auf, mit welcher sich der erste Vorsprung über eine Grundebene des ersten Schaftabschnitts hinaus erstreckt. Wenn vorhanden, weist der zweite Vorsprung eine senkrecht zu der zweiten Begrenzungsfläche gemessene zweite Höhe auf, mit welcher sich der zweite Vorsprung über eine Grundebene des zweiten Schaftabschnitts hinaus erstreckt. Die genannten Grundebenen bilden einander zugewandte Begrenzungsflächen des schlitzförmigen Zwischenraums, und zwar längs der Schaftachse gesehen in einem Bereich, in welchem kein Vorsprung angeordnet ist. Es wird vorgeschlagen, dass die erste Höhe und/oder die zweite Höhe mindestens 0,5 mm beträgt oder betragen. Dies bedeutet, dass der schlitzförmige Zwischenraum im Bereich mindestens eines Vorsprungs um mindestens 0,5 mm Verformungsweg verengt wird, und zwar im Vergleich zu einer aus dem Stand der Technik bekannten Prothese, welche nur einander zugewandte Grundebenen aufwiesen und keinen erfindungsgemäßen Vorsprung oder keine erfindungsgemäßen Vorsprünge.

Sofern zwei Vorsprünge vorgesehen sind, welche längs der Schaftachse gesehen auf derselben Höhe angeordnet sind, wird der schlitzförmige Zwischenraum im Vergleich zum Stand der Technik um mindestens 1 mm verengt, ein gemeinsamer Verformungsweg der Schaftabschnitt im Vergleich zum Stand der Technik also um mindestens 1 mm verkürzt.

Insbesondere ist es bevorzugt, wenn ein Verhältnis zwischen der ersten Höhe oder der zweiten Höhe oder zwischen der Summe aus der ersten Höhe oder der zweiten Höhe oder zwischen der Summe aus der ersten Höhe und der zweiten Höhe einerseits und dem ("vorsprungfreien") Schaftabschnittsabstand andererseits mindestens 10%, insbesondere mindestens 20% beträgt. Dies geht mit einer Verkürzung des Verformungswegs - bezogen auf den "vorsprungfreien" Stand der Technik - um mindestens 10%, insbesondere um mindestens 20%, einher.

Es ist ferner vorteilhaft, wenn der Schaft eine Umfangsfläche mit sich parallel zu der Schaftachse erstreckenden Längsnuten aufweist. Eine solche Gestaltung einer umfangsseitig geschlossenen Fläche eines Schafts kann auch dadurch beschrieben werden, dass der Schaft ein "Wagner-Profil" aufweist. Die Längsnuten unterstützen eine zuverlässige Verankerung an einer Begrenzung des Knochenmarkraums, bilden für sich genommen jedoch lokale Schwächungen des Querschnitts der Schaftabschnitte. Die erfindungsgemäße Prothese ermöglicht eine Kompensation dieser Schwächung.

Weitere Merkmale und Vorteile der Erfindung sind Gegenstand der nachfolgenden Beschreibung eines bevorzugten Ausführungsbeispiels.

In der Zeichnung zeigt:
- Fig. 1: eine Schnittdarstellung einer aus dem Stand der Technik bekannten Prothese;
- Fig. 2: eine Seitenansicht einer erfindungsgemäßen Ausführungsform einer Prothese;
- Fig. 3: einen in Fig. 2 mit III bezeichneten Ausschnitt in vergrößerter Darstellung.

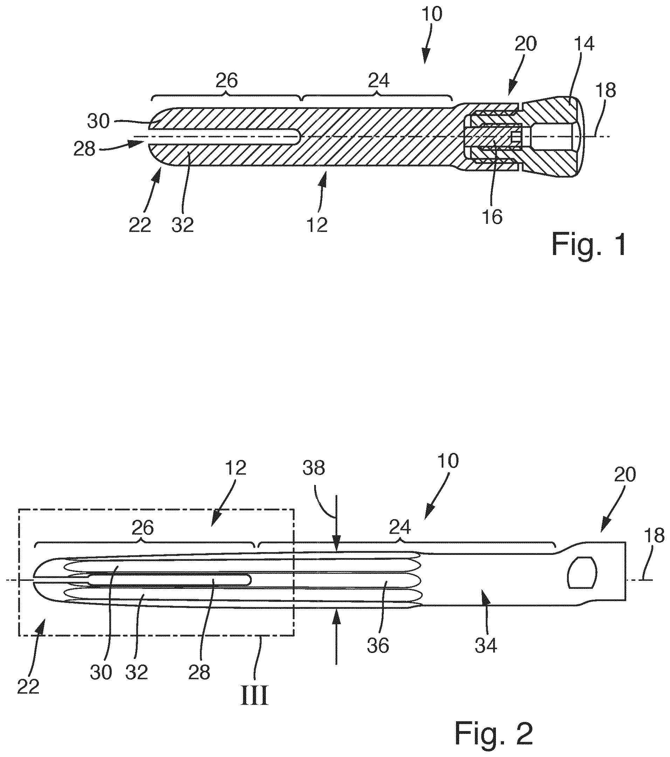

Eine aus dem Stand der Technik (EP 0 966 928 A2) bekannte Prothese ist in Figur 1 dargestellt und mit dem Bezugszeichen 10 bezeichnet. Die Prothese weist einen Schaft 12 auf, der einenends mit einem Gelenkabschnitt 14 verbunden ist, beispielsweise unter Zwischenschaltung eines Verbindungsteils 16.

Der Schaft 12 erstreckt sich längs einer Schaftachse 18 und zwar zwischen einem dem Gelenkabschnitt 14 zugewandten proximalen Ende und einem freien, distalen Ende 22, welches bei Anordnung des Schafts in einem Knochenmarkraum eines Röhrenknochens zuerst in den Knochenmarkraum eingeführt wird.

Ausgehend von dem proximalen Ende 20 weist der Schaft 12 entlang einer ersten Teillänge 24 einen ersten, insbesondere massiven Querschnitt auf. Entlang einer zweiten Teillänge 26, welche das distale Ende 22 des Schafts 12 einschließt, weist der Schaft 12 einen von dem ersten Querschnitt abweichenden zweiten Querschnitt auf.

Entlang der zweiten Teillänge 26 ist ein sich parallel zu der Schaftachse 18 erstreckender schlitzförmiger Zwischenraum 28 vorgesehen. Der Zwischenraum 28 ist auf voneinander abgewandten Seiten durch einen ersten Schaftabschnitt 30 und einen hierzu beabstandeten zweiten Schaftabschnitt 32 begrenzt.

Für die nachfolgende Beschreibung eines erfindungsgemäßen Ausführungsbeispiels einer Prothese 10 wird auf die vorstehende Beschreibung zu Figur 1 Bezug genommen. Die Prothese 10 gemäß Figur 2 kann beispielsweise als Knieprothese oder Hüftschaftprothese ausgebildet sein; es versteht sich, dass auch bei der Prothese 10 ein (nicht dargestellter) Gelenkabschnitt 14, ggf. unter Verwendung eines Zwischenteils 16, vorgesehen sein kann.

Der Schaft 12 weist eine Umfangsfläche 34 auf, welche längs der Schaftachse 18 gesehen zumindest abschnittsweise mit Längsnuten 36 versehen ist, welche über den Umfang des Schaftes 12 hinweg verteilt angeordnet sind, insbesondere regelmäßig verteilt angeordnet sind. Beispielsweise sind insgesamt zehn in Umfangsrichtung zueinander versetzte Längsnuten 36 vorgesehen.

Der Schaft 12 weist einen maximalen Durchmesser 38 auf, der beispielsweise zwischen 10 mm und 24 mm beträgt.

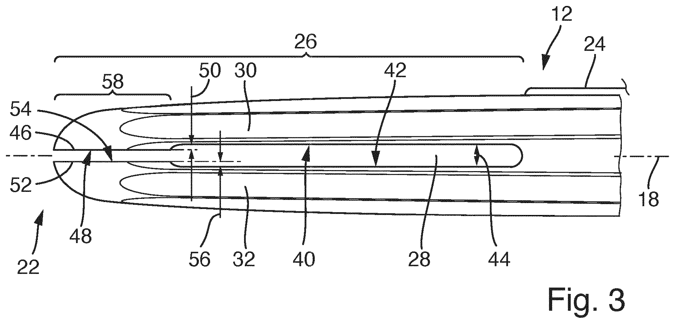

Für die weitere Beschreibung wird auf die Figur 3 Bezug genommen.

Der erste Schaftabschnitt 30 weist in einem sich an die erste Teillänge 24 des Schafts 12 anschließenden Bereich eine dem zweiten Schaftabschnitt 32 zugewandte Grundebene 40 auf. Der zweite Schaftabschnitt 32 weist eine der ersten Grundebene 40 zugewandte zweite Grundebene 42 auf. Die Grundebenen 40 und 42 sind über einen Schaftabschnittsabstand 44 hinweg zueinander beabstandet und bilden Begrenzungen des schlitzförmigen Zwischenraums 28.

Der erste Schaftabschnitt 30 weist auf seiner dem zweiten Schaftabschnitt 32 zugewandten Seite einen Vorsprung 46 auf, der eine den zweiten Schaftabschnitt 32 zugewandte erste Begrenzungsfläche 48 aufweist. Der Abstand zwischen der ersten Begrenzungsfläche 48 und der Grundebene 40 entspricht einer Höhe 50 des ersten Vorsprungs 46. Der erste Vorsprung 46 ist insbesondere mit dem ersten Schaftabschnitt 30 einstückig ausgebildet.

Der zweite Schaftabschnitt 32 trägt auf seiner dem ersten Schaftabschnitt 30 zugewandten Seite einen zweiten Vorsprung 52 mit einer dem ersten Schaftabschnitt 30 zugewandten zweiten Begrenzungsfläche 54. Der Abstand der zweiten Begrenzungsfläche 54 zu der Grundebene 42 des zweiten Schaftabschnitts 32 entspricht einer zweiten Höhe 56 des zweiten Vorsprungs 52. Der zweite Vorsprung 52 ist insbesondere mit dem ersten Schaftabschnitt 30 einstückig ausgebildet.

Die erste Höhe 50 beträgt mindestens 0,5 mm; die zweite Höhe 56 beträgt mindestens 0,5 mm.

Die Vorsprünge 46 und 52 bilden jeweilige Verengungen des schlitzförmigen Zwischenraums 28. Im Bereich der Begrenzungsflächen 46, 48 ist der schlitzförmige Zwischenraum 28 um die Summe der Höhen 50 und 56 der Vorsprünge 46 und 52 verengt.

Die Begrenzungsflächen 48 und 54 sind zueinander parallel orientiert, insbesondere in einem unverformten Ruhezustand des Schafts 12.

Die Begrenzungsflächen 48 und 54 sind bezogen auf die Schaftachse 18 auf voneinander abgewandten Seiten der Schaftachse 18 angeordnet und hinsichtlich ihrer Abmessungen und Lage bezogen auf die Schaftachse 18 zueinander spiegelsymmetrisch.

Die Begrenzungsflächen 48 und 54 erstrecken sich längs der Schaftachse 18 hinweg über eine vorzugsweise identische Begrenzungsflächenlänge 58 hinweg.

Ausgehend von dem in der Zeichnung dargestellten unverformten Ruhezustand der Schaftabschnitte 30 und 32 kann das distale Ende 22 in einen Knochenmarkraum eines Röhrenknochens, insbesondere eines Schienbeins oder eines Oberschenkelknochens, eingeführt werden. Dabei ist der Durchmesser 38 des Schafts 12 im Verhältnis zu dem Durchmesser des Knochenmarkraums so ausgewählt, dass der Schaft vorzugsweise über die Erstreckung der Längsnuten 36 hinweg in den Knochenmarkraum einführbar ist, und dass nach dieser Einführung die nach radial außen weisenden Flächen der Schaftabschnitte 30 und 32 unter Vorspannung an jeweiligen Begrenzungsabschnitten des Knochenmarkraums anliegen.

Ausgehend von einem unverformten Ausgangszustand, siehe Figur 3, können die Schaftabschnitte 30 und 32 biegeverformt werden, wobei sich die Begrenzungsflächen 48 und 54 aufeinander zu bewegen, und zwar bis hin zu einem Zustand, in welchem die Begrenzungsflächen 48 und 54 zumindest abschnittsweise, vorzugsweise flächig, aneinander anliegen. Diese Anlage definiert einen maximalen Verformungsweg der Schaftabschnitte 30 und 32 über die Begrenzungsflächenlänge 58 hinweg.

Bei Annahme eines zu dem Stand der Technik (siehe Figur 1) identischen Schaftabschnittsabstands 44 und bei Annahme identischer Materialien und einer identischen Dimensionierung der Schaftabschnitte 30 und 32 und bei Annahme einer identischen zweiten Teillänge 26 wird der Verformungsweg der Schaftabschnitte 30 und 32 im Bereich des distalen Endes 28 also um ein Maß verkürzt, dass der Summe der Höhen 50 und 56 der Vorsprünge 46 und 52 entspricht. Auf diese Weise wird erreicht, dass die Schaftabschnitte 30 und 32 mit niedrigen Verformungskräften biegeverformbar sind und in entsprechender Weise nach dem Einsetzen in einen Röhrenknochen nur niedrige Rückstellkräfte entwickeln und gleichzeitig eine unerwünschte plastische Verformung oder ein Versagen mit Bruch der Schaftabschnitte 30 und 32 verhindert wird.

## Patentansprüche

1. Prothese (10), insbesondere Knieprothese oder Hüftschaftprothese, mit einem Schaft (12) zur Anordnung und Befestigung in einem Röhrenknochen, wobei der Schaft (12) sich längs einer Schaftachse (18) zwischen einem proximalen Ende (20) und einem in den Röhrenknochen einführbaren distalen Ende (22) erstreckt, wobei der Schaft (12) von dem proximalen Ende (20) ausgehend entlang einer ersten Teillänge (24) einen ersten, insbesondere massiven, Querschnitt aufweist und wobei der Schaft (12) entlang einer zweiten Teillänge (26), welche das distale Ende (22) des Schafts (12) einschließt, einen von dem ersten Querschnitt abweichenden zweiten Querschnitt aufweist, wobei der Schaft (12) entlang der zweiten Teillänge (26) sich parallel zu der Schaftachse (18) erstreckende Schaftabschnitte (30, 32) aufweist, welche durch einen schlitzförmigen Zwischenraum (28) voneinander getrennt sind, wobei die Schaftabschnitte (30, 32) ausgehend von einem unverformten Ruhezustand durch Biegebelastung derart verformbar sind, dass sich der schlitzförmige Zwischenraum (28) verkleinert, **dadurch gekennzeichnet, dass** ein erster Schaftabschnitt (30) einen ersten Vorsprung (46) aufweist, der auf einer einem zweiten Schaftabschnitt (32) zugewandten Seite des ersten Schaftabschnitt (30) vorgesehen ist, eine erste Verengung des Zwischenraums (28) bildet und entlang der Schaftachse (18) gesehen zu der ersten Teillänge (24) des Schafts (12) beabstandet angeordnet ist, wobei der erste Vorsprung (46) eine erste Begrenzungsfläche (48) zur Anlage an dem zweiten Schaftabschnitt (32) aufweist, wobei die erste Begrenzungsfläche (48) in dem unverformten Ruhezustand zu dem zweiten Schaftabschnitt (32) beabstandet ist.

2. Prothese (10) nach Anspruch 1, **dadurch gekennzeichnet, dass** der zweite Schaftabschnitt (32) einen zweiten Vorsprung (52) aufweist, der auf einer dem ersten Schaftabschnitt (30) zugewandten Seite des zweiten Schaftabschnitt (32) vorgesehen ist, eine zweite Verengung des Zwischenraums (28) bildet und entlang der Schaftachse (18) gesehen zu der ersten Teillänge (24) des Schafts (12) beabstandet angeordnet ist, wobei der zweite Vorsprung (52) eine zweite Begrenzungsfläche (54) zur Anlage an dem ersten Schaftabschnitt (30) aufweist, wobei die zweite Begrenzungsfläche (54) in dem unverformten Ruhezustand zu dem ersten Schaftabschnitt (30) beabstandet ist.

3. Prothese (10) nach Anspruch 2, **dadurch gekennzeichnet, dass** eine Biegeverformung der Schaftabschnitte (30, 32) im Bereich des ersten Vorsprungs (46) und des zweiten Vorsprungs (52) durch eine Anlage der ersten Begrenzungsfläche (48) und der zweiten Begrenzungsfläche (54) begrenzt ist.

4. Prothese (10) nach Anspruch 3, **dadurch gekennzeichnet, dass** in dem unverformten Ruhezustand der Schaftabschnitte (30, 32) oder in einem verformten Zustand der Schaftabschnitte (30, 32) die erste Begrenzungsfläche (48) und die zweite Begrenzungsfläche (54) zueinander parallel orientiert sind.

5. Prothese (10) nach einem der vorstehenden Ansprüche, **dadurch gekennzeichnet, dass** sich die erste Begrenzungsfläche (48) und/oder die zweite Begrenzungsfläche (54) längs der Schaftachse (18) gesehen bis zu dem distalen Ende (22) des Schafts (12) erstreckt oder erstrecken.

6. Prothese (10) nach einem der vorstehenden Ansprüche, **dadurch gekennzeichnet, dass** die erste Begrenzungsfläche (48) und/oder die zweite Begrenzungsfläche (54) längs der Schaftachse (18) gesehen eine Begrenzungsflächenlänge (58) aufweist oder aufweisen, welche zwischen 5% und 45%, insbesondere zwischen 15% und 35%, der zweiten Teillänge (26) beträgt.

7. Prothese (10)nach einem der vorstehenden Ansprüche, **dadurch gekennzeichnet, dass** die Schaftabschnitte (30, 32) in einem sich an die erste Teillänge (24) des Schafts (12) anschließenden Bereich der zweiten Teillänge (26) des Schafts (12) und in dem unverformten Ruhezustand der Schaftabschnitte (30, 32) einen der Breite des schlitzförmigen Zwischenraums (28) entsprechenden Schaftabschnittsabstand (44) zueinander aufweisen, vorzugsweise wobei der Schaftabschnittsabstand (44) mindestens 20%, insbesondere mindestens 25%, eines maximalen Durchmessers (38) einer Umfangsfläche (34) des Schafts (12) beträgt.

8. Prothese (10) nach einem der vorstehenden Ansprüche, **dadurch gekennzeichnet, dass** der erste Vorsprung (46) eine senkrecht zu der ersten Begrenzungsfläche (48) gemessene erste Höhe (50) aufweist und/oder dass der zweite Vorsprung (52) eine senkrecht zu der zweiten Begrenzungsfläche (54) gemessene zweite Höhe (56) aufweist, vorzugsweise wobei die erste Höhe (50) und/oder die zweite Höhe (56) mindestens 0,5 mm beträgt oder betragen.

9. Prothese (10) nach Anspruch 8 bei Rückbezug auf Anspruch 7, **dadurch gekennzeichnet, dass** ein Verhältnis zwischen der ersten Höhe (50) oder der zweiten Höhe (56) oder zwischen der Summe aus der ersten Höhe (50) und der zweiten Höhe (56) einerseits und dem Schaftabschnittsabstand (44) andererseits mindestens 10%, insbesondere mindestens 20%, beträgt.

10. Prothese (10) nach einem der vorstehenden Ansprüche, **dadurch gekennzeichnet, dass** der Schaft (12) eine Umfangsfläche (34) mit sich parallel zu der Schaftachse (18) erstreckenden Längsnuten (36) aufweist.

## Claims

1. Prosthesis (10), in particular a knee prosthesis or a hip stem prosthesis, comprising a shaft (12) to be arranged and fastened in a long bone, the shaft (12) extending along a shaft axis (18) between a proximal end (20) and a distal end (22) that can be inserted into the long bone, the shaft (12), along a first partial length (24) starting at the proximal end (20), having a first in particular solid cross section, and the shaft (12), along a second partial length (26) that includes the distal end (22) of the shaft (12), having a second cross section that differs from the first cross section, the shaft (12), along the second partial length (26), having shaft portions (30, 32) extending parallel to the shaft axis (18), which shaft portions are separated from each other by a slot-shaped interspace (28), it being possible for the shaft portions (30, 32), starting from an undeformed rest state, to be deformed by bending load in such a way that the slot-shaped interspace (28) decreases in size,
**characterized in that** a first shaft portion (30) has a first projection (46) which is provided on a side of the first shaft portion (30) facing a second shaft portion (32), forms a first narrowing of the interspace (28) and is arranged spaced apart from the first partial length (24) of the shaft (12), considered along the shaft axis (18), the first projection (46) having a first boundary surface (48) for bearing against the second shaft portion (32), the first boundary surface (48) being spaced apart from the second shaft portion (32) in the undeformed rest state.

2. Prosthesis (10) according to claim 1, **characterized in that** the second shaft portion (32) has a second projection (52) which is provided on a side of the second shaft portion (32) facing the first shaft portion (30), forms a second narrowing of the interspace (28) and is arranged spaced apart from the first partial length (24) of the shaft (12), considered along the shaft axis (18), the second projection (52) having a second boundary surface (54) for bearing against the first shaft portion (30), the second boundary surface (54) being spaced apart from the first shaft portion (30) in the undeformed rest state.

3. Prosthesis (10) according to claim 2, **characterized in that** a bending deformation of the shaft portions (30, 32) in the region of the first projection (46) and of the second projection (52) is limited by virtue of said projections bearing against the first boundary surface (48) and the second boundary surface (54).

4. Prosthesis (10) according to claim 3, **characterized in that** in the undeformed rest state of the shaft portions (30, 32) or in a deformed state of the shaft portions (30, 32), the first boundary surface (48) and the second boundary surface (54) are oriented parallel to each other.

5. Prosthesis (10) according to any of the preceding claims,
**characterized in that** the first boundary surface (48) and/or the second boundary surface (54) extend or extends to the distal end (22) of the shaft (12), considered along the shaft axis (18).

6. Prosthesis (10) according to any of the preceding claims,
**characterized in that** the first boundary surface (48) and/or the second boundary surface (54) have or has a boundary surface length (58), considered along the shaft axis (18), of between 5% and 45%, in particular between 15% and 35%, of the second partial length (26).

7. Prosthesis (10) according to any of the preceding claims,
**characterized in that** the shaft portions (30, 32), in a region of the second partial length (26) of the shaft (12) adjoining the first partial length (24) of the shaft (12) and in the undeformed rest state of the shaft portions (30, 32), have a shaft portion spacing (44) relative to each other which corresponds to the width of the slot-shaped interspace (28), the shaft portion spacing (44) preferably being at least 20%, in particular at least 25%, of a maximum diameter (38) of a circumferential surface (34) of the shaft (12).

8. Prosthesis (10) according to any of the preceding claims,
**characterized in that** the first projection (46) has a first height (50) measured perpendicular to the first boundary surface (48), **and/or in that** the second projection (52) has a second height (56) measured perpendicular to the second boundary surface (54), the first height (50) and/or the second height (56) preferably being at least 0.5 mm.

9. Prosthesis (10) according to claim 8 when dependent on claim 7,
**characterized in that** a ratio between the first height (50) or the second height (56) or between the sum of the first height (50) and the second height (56) on the one hand and the shaft portion spacing (44) on the other hand is at least 10%, in particular at least 20%.

10. Prosthesis (10) according to any of the preceding claims,
**characterized in that** the shaft (12) has a circumferential surface (34) comprising longitudinal grooves (36) extending parallel to the shaft axis (18).

## Revendications

1. Prothèse (10), en particulier prothèse de genou ou prothèse de tige de hanche, comportant une tige (12) pour l'agencement et la fixation dans un os tubulaire, dans laquelle la tige (12) s'étend le long d'un axe de tige (18) entre une extrémité proximale (20) et une extrémité distale (22) pouvant être introduite dans l'os tubulaire, dans laquelle la tige (12) présente une première section transversale, en particulier massive, le long d'une première longueur partielle (24) à partir de l'extrémité proximale (20), et dans laquelle la tige (12) présente une seconde section transversale différente de la première section transversale le long d'une seconde longueur partielle (26) qui inclut l'extrémité distale (22) de la tige (12), dans laquelle la tige (12) présente le long de la seconde longueur partielle (26) des sections de tige (30, 32) s'étendant parallèlement à l'axe de tige (18) et séparées les unes des autres par un espace intermédiaire (28) en forme de fente, dans laquelle les sections de tige (30, 32) peuvent être déformées, à partir d'un état de repos non déformé, par une charge de flexion de telle sorte que l'espace intermédiaire (28) en forme de fente se rétrécit, **caractérisée en ce qu'**une première section de tige (30) présente une première saillie (46) qui est prévue sur un côté de la première section de tige (30) tourné vers une seconde section de tige (32), forme un premier rétrécissement de l'espace intermédiaire (28) et, vue le long de l'axe de tige (18), est disposée à distance de la première longueur partielle (24) de la tige (12), dans laquelle la première saillie (46) présente une première surface de délimitation (48) destinée à s'appuyer contre la seconde section de tige (32), dans laquelle la première surface de délimitation (48) est espacée de la seconde section de tige (32) dans l'état de repos non déformé.

2. Prothèse (10) selon la revendication 1, **caractérisée en ce que** la seconde section de tige (32) présente une seconde saillie (52) qui est prévue sur un côté de la seconde section de tige (32) tourné vers la première section de tige (30), forme un second rétrécissement de l'espace intermédiaire (28) et est disposée à distance de la première longueur partielle (24) de la tige (12), vue le long de l'axe de tige (18), dans laquelle la seconde saillie (52) présente une seconde surface de délimitation (54) destinée à s'appuyer contre la première section de tige (30), dans laquelle la seconde surface de délimitation (54) est espacée de la première section de tige (30) dans l'état de repos non déformé.

3. Prothèse (10) selon la revendication 2, **caractérisée en ce qu'**une déformation en flexion des sections de tige (30, 32) dans la zone de la première saillie (46) et de la seconde saillie (52) est limitée par un appui de la première surface de délimitation (48) et de la seconde surface de délimitation (54).

4. Prothèse (10) selon la revendication 3, **caractérisée en ce que,** dans l'état de repos non déformé des sections de tige (30, 32) ou dans un état déformé des sections de tige (30, 32), la première surface de délimitation (48) et la seconde surface de délimitation (54) sont orientées parallèlement l'une à l'autre.

5. Prothèse (10) selon l'une des revendications précédentes, **caractérisée en ce que** la première surface de délimitation (48) et/ou la seconde surface de délimitation (54), vues le long de l'axe de tige (18), s'étendent jusqu'à l'extrémité distale (22) de la tige (12).

6. Prothèse (10) selon l'une des revendications précédentes, **caractérisée en ce que** la première surface de délimitation (48) et/ou la seconde surface de délimitation (54), vues le long de l'axe de tige (18), présentent une longueur de surface de délimitation (58) qui est comprise entre 5 % et 45 %, en particulier entre 15 % et 35 %, de la seconde longueur partielle (26).

7. Prothèse (10) selon l'une des revendications précédentes, **caractérisée en ce que** les sections de tige (30, 32), dans une zone de la seconde longueur partielle (26) de la tige (12) se raccordant à la première longueur partielle (24) de la tige (12) et dans l'état de repos non déformé des sections de tige (30, 32), présentent entre elles une distance entre sections de tige (44) correspondant à la largeur de l'espace intermédiaire (28) en forme de fente, de préférence dans laquelle la distance entre sections de tige (44) est d'au moins 20 %, en particulier d'au moins 25 %, d'un diamètre maximal (38) d'une surface périphérique (34) de la tige (12).

8. Prothèse (10) selon l'une des revendications précédentes, **caractérisée en ce que** la première saillie (46) présente une première hauteur (50) mesurée perpendiculairement à la première surface de délimitation (48) **et/ou en ce que** la seconde saillie (52) présente une seconde hauteur (56) mesurée perpendiculairement à la seconde surface de délimitation (54), de préférence dans laquelle la première hauteur (50) et/ou la seconde hauteur (56) sont d'au moins 0,5 mm.

9. Prothèse (10) selon la revendication 8 en référence à la revendication 7, **caractérisée en ce qu'un** rapport entre la première hauteur (50) ou la seconde hauteur (56) ou entre la somme de la première hauteur (50) et de la seconde hauteur (56) d'une part et la distance entre sections de tige (44) d'autre part est d'au moins 10 %, en particulier d'au moins 20 %.

10. Prothèse (10) selon l'une des revendications précédentes, **caractérisée en ce que** la tige (12) présente une surface périphérique (34) comportant des rainures longitudinales (36) s'étendant parallèlement à l'axe de tige (18).
